(19) Europäisches Patentamt
European Patent Office
Office européen des brevets

(11) Veröffentlichungsnummer: **0 551 831 A1**

# (12) EUROPÄISCHE PATENTANMELDUNG

(21) Anmeldenummer: **93100148.1**

(22) Anmeldetag: **07.01.93**

(51) Int. Cl.5: **C07D 213/84**, C07D 215/48, C07D 217/26, C07D 221/04

(30) Priorität: **15.01.92 DE 4200820**

(43) Veröffentlichungstag der Anmeldung: **21.07.93 Patentblatt 93/29**

(84) Benannte Vertragsstaaten: **AT BE CH DE DK ES FR GB IT LI NL SE**

(71) Anmelder: **HOECHST AKTIENGESELLSCHAFT**
**Postfach 80 03 20**
**W-6230 Frankfurt am Main 80(DE)**

(72) Erfinder: **Giencke, Wolfgang, Dr.**
**Am Steinberg 45**
**W-6238 Hofheim/Ts.(DE)**
Erfinder: **Vermehren, Jan, Dr.**
**Im Hinterlenzen 33**
**W-6270 Idstein/Taunus(DE)**

## (54) Verfahren zur Herstellung substituierter 2-Cyanopyridine.

(57) Verbindungen der allgemeinen Formel I

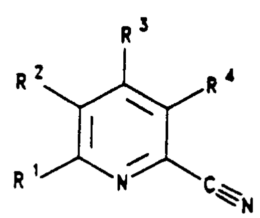

I

worin

- $R^1$ H, Alkyl, Alkenyl, Alkinyl, Haloalkyl, Alkoxycarbonyl, Alkoxyalkyl, Phenyl, Benzyl, Phenoxyalkyl bedeutet, wobei die drei letztgenannten Reste im Phenylteil substituiert sein können;
- $R^2$ H, Alkyl, Alkenyl, Alkinyl, Haloalkyl oder Alkoxycarbonyl bedeutet;
- $R^3$ H, Alkyl, Alkenyl, Alkinyl, Haloalkyl, Alkoxycarbonyl, Phenyl, Phenoxy, Benzyl, wobei die drei letztgenannten Reste substituiert sein können, oder Alkoxy bedeutet; und
- $R^4$ H, Alkyl, Alkenyl, Alkinyl, Haloalkyl oder Alkoxycarbonyl bedeutet;

oder

- $R^1$ gemeinsam mit $R^2$ für eine gesättigte Kette steht und die übrigen Reste wie oben definiert sind;

oder

- $R^1$ gemeinsam mit $R^2$ für eine Kette der Formel -CH=CH-CH=CH- steht, welche substituiert sein kann und die übrigen Reste wie oben definiert sind;

oder

- $R^3$ gemeinsam mit $R^4$ für eine Kette der Formel -CH=CH-CH=CH- steht, welche substituiert sein kann und die übrigen Reste wie oben definiert sind;

sind wertvolle Zwischenprodukte bei der Herstellung von Arzneimitteln und Pflanzenschutzmitteln.

Im erfindungsgemäßen Verfahren erhält man Verbindungen der Formel I, indem man ein substituiertes 1-Alkoxypyridiniumsalz der allgemeinen Formel II

EP 0 551 831 A1

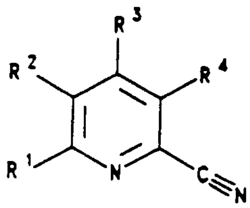

I

in welcher
- $R^1$ bis $R^4$ die gleiche Bedeutung wie in Formel I haben und
- $R^5$ Alkyl, Alkenyl, Alkinyl, Benzyl oder Trialkylsilyl bedeutet und
- X eine Abgangsgruppe bedeutet;

mit einem Cyanid der allgemeinen Formel III

$M(CN)_m$ (III),

in welcher
- M Alkali, Erdalkali oder Kupfer bedeutet und
- m 1 oder 2 ist,

in einem polaren aprotischen Lösungsmittel oder Lösungsmittelgemisch miteinander umsetzt.

Die Erfindung betrifft ein Verfahren zur Herstellung substituierter 2-Cyanopyridine durch Umsetzung entsprechender 1-Alkoxypyridiniumsalze mit Metallcyaniden in einem polaren aprotischen Lösungsmittel oder einem Gemisch solcher Lösungsmittel. Substituierte 2-Cyanopyridine sind wertvolle Zwischenprodukte bei der Herstellung von Arzneimitteln und Pflanzenschutzmitteln, wie sie beispielsweise in der deutschen Patentanmeldung P 40 32 878.3 vorgeschlagen worden sind.

Substituierte 2-Cyanopyridine wurden bisher im wesentlichen nach zwei Verfahren hergestellt. Man setzte entweder ein 1-Alkoxypyridiniumsalz mit einem Alkalicyanid in Wasser um oder brachte Pyridin-N-oxide in organischen Lösungsmitteln mit Trialkylsilylcyaniden unter Zuhilfenahme eines Acylierungsmittels wie N,N-Dimethylcarbaminsäurechlorid zur Reaktion.

Beide Verfahren besitzen erhebliche Nachteile.

So ist zum Beispiel im ersten Verfahren ein großer Überschuß an Alkalicyanid notwendig (Org. Synth. Coll. Vol. V, 269), was die Aufarbeitung erschwert und gefährlich macht. Die Ausbeuten bei diesem Verfahren sind in vielen Fällen unbefriedigend, da mehrere Isomere, insbesondere auch 4-Cyanopyridine, entstehen können und die Trennung kompliziert ist.

Ein zweites Verfahren weist als Nachteil die Notwendigkeit des stöchiometrischen Einsatzes des teuren und hochgiftigen Trimethylsilylcyanides sowie des cancerogenen N,N-Dimethylcarbaminsäurechlorides auf (J. Org. Chem. 48, 1375 [1983]). Beide Reagenzien eignen sich aufgrund ihrer Eigenschaften und des daraus resultierenden Gefährdungspotentials nicht für die Herstellung größerer Mengen der 2-Cyanopyridine.

Überraschend wurde nun gefunden, daß substituierte 1-Alkoxypyridiniumsalze mit Cyaniden ausgezeichnet in einem polaren aprotischen Lösungsmittel wie z.B. Dimethylformamid, Dimethylacetamid, N-Methylpyrrolidon, N,N'-Dimethylpropylenharnstoff, HMPT, Aceton, Acetonitril, Propionitril, Diethylenglykoldimethylether (Diglyme), Tetraglyme, Tetrahydrofuran, Dimethylsulfoxid oder Sulfolan, oder einer Mischung aus zwei oder mehr Komponenten unter Bildung von 2-Cyanopyridinen reagieren, wobei die Ausgangsverbindungen in nahezu äquimolaren Verhältnissen eingesetzt werden können.

Die Erfindung betrifft vorzugsweise ein Verfahren zur Herstellung von Verbindungen der allgemeinen Formel I

(I) 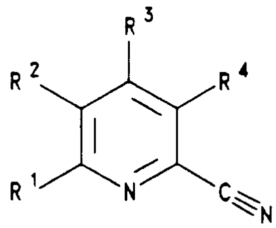

in welcher

- $R^1$ H, Alkyl, Alkenyl, Alkinyl, Haloalkyl, Alkoxycarbonyl, Alkoxyalkyl, Phenyl, Benzyl, Phenoxyalkyl bedeutet, wobei die drei letztgenannten Reste im Phenylteil substituiert sein können;
- $R^2$ H, Alkyl, Alkenyl, Alkinyl, Haloalkyl oder Alkoxycarbonyl bedeutet;
- $R^3$ H, Alkyl, Alkenyl, Alkinyl, Haloalkyl, Alkoxycarbonyl, Phenyl, Phenoxy, Benzyl, wobei die drei letztgenannten Reste substituiert sein können, oder Alkoxy bedeutet; und
- $R^4$ H, Alkyl, Alkenyl, Alkinyl, Haloalkyl oder Alkoxycarbonyl bedeutet;

oder

- $R^1$ gemeinsam mit $R^2$ für eine gesättigte Kette steht und die übrigen Reste wie oben definiert sind;

oder

- $R^1$ gemeinsam mit $R^2$ für eine Kette der Formel -CH=CH-CH=CH- steht, welche substituiert sein kann und die übrigen Reste wie oben definiert sind;

oder

- $R^3$ gemeinsam mit $R^4$ für eine Kette der Formel -CH=CH-CH=CH- steht, welche substituiert sein kann und die übrigen Reste wie oben definiert sind;

das dadurch gekennzeichnet ist, daß man ein substituiertes 1-Alkoxypyridiniumsalz der allgemeinen Formel II,

(II) 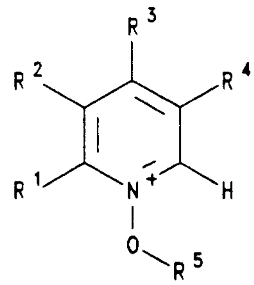 $X^-$

in welcher

$R^1$ bis $R^4$ die gleiche Bedeutung wie in Formel I haben und

$R^5$ Alkyl, Alkenyl, Alkinyl, Benzyl oder Trialkylsilyl bedeutet und

X eine Abgangsgruppe bedeutet;

mit einem Cyanid der allgemeinen Formel III

$M(CN)_m$ (III),

in welcher

M Alkali, Erdalkali oder Kupfer bedeutet und

m 1 oder 2 ist,

in einem polaren aprotischen Lösungsmittel oder Lösungsmittelgemisch miteinander umsetzt.

Die Erfindung betrifft insbesondere ein Verfahren zur Herstellung substituierter 2-Cyanopyridine der allgemeinen Formel I, in welcher

$R^1$ H, $C_1$-$C_6$-Alkyl, vorzugsweise $C_1$-$C_4$-Alkyl, $C_2$-$C_6$-Alkenyl, $C_2$-$C_6$-Alkinyl, $C_1$-$C_4$-Haloalkyl, $C_1$-$C_6$-Alkoxycarbonyl, $C_1$-$C_4$-Alkoxy-$C_1$-$C_4$-alkyl, Phenyl, Benzyl, Phenoxy-$C_1$-$C_2$-alkyl bedeutet, wobei die drei letztgenannten Reste im Phenylteil bis zu fünffach, vorzugsweise bis zu dreifach durch gleiche oder verschiedene Reste aus der Reihe $C_1$-$C_4$-Alkyl, vorzugsweise $C_1$-$C_2$-Alkyl, Halogen, $C_1$-$C_4$-Alkoxy, vorzugsweise $C_1$-$C_2$-Alkoxy, $C_1$-$C_4$-Haloalkyl und $C_1$-$C_2$-Haloalkoxy substituiert sein können;

$R^2$ H, $C_1$-$C_6$-Alkyl, vorzugsweise $C_1$-$C_4$-Alkyl, $C_2$-$C_6$-Alkenyl, $C_2$-$C_6$-Alkinyl, $C_1$-$C_4$-Haloalkyl oder $C_1$-$C_6$-Alkoxycarbonyl bedeutet;

$R^3$ H, $C_1$-$C_6$-Alkyl, vorzugsweise $C_1$-$C_4$-Alkyl, $C_2$-$C_6$-Alkenyl, $C_2$-$C_6$-Alkinyl, $C_1$-$C_4$-Haloalkyl, $C_1$-$C_6$-Alkoxycarbonyl, Phenyl, Phenoxy, Benzyl, wobei die drei letztgenannten Reste bis zu dreifach durch gleiche oder verschiedene Reste aus der Reihe $C_1$-$C_4$-Alkyl, vorzugsweise $C_1$-$C_2$-Alkyl, Halogen, $C_1$-$C_4$-Alkoxy, vorzugsweise $C_1$-$C_2$-Alkoxy, $C_1$-$C_4$-Haloalkyl und $C_1$-$C_2$-Haloalkoxy substituiert sein können, oder $C_1$-$C_4$-Alkoxy bedeutet; und

$R^4$ H, $C_1$-$C_6$-Alkyl, vorzugsweise $C_1$-$C_4$-Alkyl, $C_2$-$C_6$-Alkenyl, $C_2$-$C_6$-Alkinyl, $C_1$-$C_4$-Haloalkyl oder $C_1$-$C_6$-Alkoxycarbonyl bedeutet;

oder

$R^1$ gemeinsam mit $R^2$ für $-(CH_2-)_n$ steht, wobei n 2 bis 14, vorzugsweise 3 bis 12 ist und die übrigen Reste wie oben definiert sind;

oder

$R^1$ gemeinsam mit $R^2$ für eine Kette der Formel -CH=CH-CH=CH- steht, welche mit bis zu zwei gleichen oder verschiedenen Resten aus der Reihe $C_1$-$C_4$-Alkyl, vorzugsweise $CH_3$, Halogen, Phenoxy und $C_1$-$C_4$-Alkoxy, vorzugsweise $C_1$-$C_2$-Alkoxy, substituiert sein kann und die übrigen Reste wie oben definiert sind;

oder

$R^3$ gemeinsam mit $R^4$ für eine Kette der Formel -CH=CH-CH=CH- steht, welche mit bis zu zwei gleichen oder verschiedenen Resten aus der Reihe $C_1$-$C_4$-Alkyl, vorzugsweise $CH_3$, Halogen und $C_1$-$C_4$-Alkoxy, vorzugsweise $C_1$-$C_2$-Alkoxy, substituiert sein kann und die übrigen Reste wie oben definiert sind;

das dadurch gekennzeichnet ist, daß man ein substituiertes 1-Alkoxypyridiniumsalz der allgemeinen Formel II, in welcher

$R^1$ bis $R^4$ die gleiche Bedeutung wie in Formel I haben und

$R^5$ $C_1$-$C_6$-Alkyl, $C_2$-$C_4$-Alkenyl, $C_2$-$C_4$-Alkinyl, Benzyl oder Tri-$C_1$-$C_6$-alkylsilyl bedeutet und

X eine Abgangsgruppe, wie Halogen, $C_1$-$C_6$-Alkylsulfonyloxy, $C_6$-$C_{12}$-Arylsulfonyloxy, vor-

zugsweise Phenylsulfonyloxy, wobei der letztgenannte Rest im Arylteil bis zweifach durch gleiche oder verschiedene Reste Halogen oder $C_1$-$C_2$-Alkyl, vorzugsweise $CH_3$, substituiert sein kann, $C_1$-$C_4$-Haloalkylsulfonyloxy, vorzugsweise $CF_3SO_3$, $C_1$-$C_4$-Alkoxysulfonyloxy, vorzugsweise $C_1$-$C_2$-Alkyloxysulfonyloxy, bedeutet;

mit einem Cyanid der allgemeinen Formel III, in welcher

M Alkali, Erdalkali oder Kupfer, vorzugsweise Na oder K bedeutet und

m 1 oder 2 ist,

in einem polaren aprotischen Lösungsmittel oder Lösungsmittelgemisch miteinander umsetzt.

Hier und im folgenden bedeutet Halogen Fluor, Chlor, Brom oder Iod; Alkyl, Alkenyl und Alkinyl können geradkettig oder verzweigt sein, entsprechendes gilt für die Alkylteile der substituierten Reste; die Mehrfachbindungen in Alkenyl und Alkinyl können endständig oder mittelständig sein, sie können isoliert oder konjugiert sein; Alkali bedeutet hier und im folgenden Li, Na, K, Rb, Cs; Erdalkali bedeutet hier und im folgenden Mg, Ca, Sr, Ba; die Vorsilbe "Halo" bedeutet hier wie im folgenden, daß der entsprechende Rest ganz oder teilweise durch Halogen substituiert ist. Als Beispiele, nicht aber einschränkend seien genannt: $CF_3$, $CHF_2$, $C_4F_9$, $CCl_3$.

Das erfindungsmäßige Verfahren gleicht die Nachteile der oben genannten literaturbekannten Verfahren aus und stellt somit einen erheblichen technischen und wirtschaftlichen Vorteil dar. Es gestattet, Verbindungen der allgemeinen Formel I in ihrer ganzen Variationsbreite ausgehend von leicht zugänglichen Verbindungen der Formel II (Org. Synth. Coll. Vol. V, 269)in wesentlich besseren Ausbeuten, sehr viel günstigerer Regioselektivität und wesentlich verbesserter sicherheitstechnischer Handhabung herzustellen.

Das erfindungsmäßige Verfahren wird im Temperaturbereich von -78 °C bis zum Siedepunkt des Lösungsmittels, vorzugsweise im Temperaturbereich von -50 bis 70 °C, insbesondere im Temperaturbereich von -30 bis 30 °C so durchgeführt, daß trockenes, feingepulvertes Cyanid der Formel III in einem Teil des gewünschten Lösungsmittels bei der Reaktionstemperatur vorgelegt und intensiv gerührt wird. Eine Verbindung der allgemeinen Formel II wird in dem Rest des Lösungsmittels gelöst. Diese Lösung wird so zugetropft, daß die Temperatur konstant bleibt. Nach Ende der Zugabe wird bei der gewünschten Temperatur bis zum Zeitpunkt des vollständigen Umsatzes gerührt. Dieser Zeitpunkt läßt sich mit gängigen Methoden, z.B. durch dünnschichtchromatographische (DC) Untersuchung der Reaktionsmischung feststellen.

Um einen vollständigen Umsatz der Komponente der allgemeinen Formel II zu erzielen, ist es ratsam, einen geringen Überschuß der Komponente mit der allgemeinen Formel III zu verwenden. Der Überschuß an dieser Komponente beträgt 1 bis 20 mol%, vorzugsweise 2 bis 10 mol%, insbesondere 2 bis 5 mol%.

Nach beendeter Reaktion läßt man auf Raumtemperatur kommen und entfernt destillativ, gegebenenfalls unter vermindertem Druck, einen Teil des Lösungsmittels. Der Rückstand wird destilliert oder auf Wasser gegeben und mit einem geeigneten Lösungsmittel extrahiert. Nach Entfernen des Lösungsmittels wird das Endprodukt der allgemeinen Formel I in der Regel in Ausbeuten von über 90 % erhalten. Es kann durch gängige Reinigungsmethoden wie z.B. Destillation, Umkristallisation oder Chromatographie,gereinigt werden.

Zur Vermeidung von Nebenreaktionen durch Luftsauerstoff oder Feuchtigkeit wird das erfindungsmäßige Verfahren vorzugsweise unter einer Schutzgasatmosphäre durchgeführt. Als Schutzgase kommen z.B. Stickstoff oder Argon in Betracht.

Die folgenden Beispiele sollen zur Erläuterung der Erfindung dienen, ohne das diese darauf beschränkt wäre.

Beispiel 1:

2-Cyano-6-methylpyridin (Tabellenbeispiel Nr. 01)

In einem 10 l-Kolben wurden 516 g (10,5 mol) trockenes, pulverisiertes Natriumcyanid in 3 l Dimethylformamid vorgelegt. Zu diesem Gemisch wurde unter intensivem Rühren bei 25 °C eine Lösung von 2350 g (10,3 mol) 1-Methoxy-2-methylpyridinium-methylsulfat in 1,5 l Dimethylformamid getropft. Nach vollständiger Zugabe ließ man 24 Stunden bei Raumtemperatur rühren und saugte anschließend die unlöslichen Bestandteile ab. Das Filtrat wurde im Vakuum eingeengt und das Rohprodukt über eine kurze Kolonne destilliert. Das Destillat erstarrte zu weißen Nadeln (Fp: 72 - 73 °C); Ausbeute: 1150 g (94,6 % d.Th.). Sdp.: 115-117 °C/0,1 mbar)

Beispiel 2:

2-Cyano-5,6-cyclohexenopyridin (Tabellenbeispiel Nr. 51)

51,5 g (1,05 mol) Natriumcyanid, die zuvor durch 12-stündiges Stehen im Vakuum bei 80 °C getrocknet wurden, wurden in 300 ml trockenem Dimethylformamid unter Argon suspendiert. Es wurde auf -20 °C abgekühlt und unter Argon eine Lösung von 275,3 g (1,00 mol) 5,6-Cyclohexeno-N-methoxypyridinium-methylsulfat in 300 ml Dimethylformamid so zugetropft, daß die Temperatur konstant blieb. Nach beendeter Zugabe wurde bis zum vollständigen Umsatz weitergerührt und man ließ auf Raumtemperatur kommen. Das Lösungsmittel wurde vollständig unter vermindertem Druck entfernt und der Rückstand auf Wasser gegossen. Es wurde mehrfach mit Ethylacetat extrahiert und die organischen Lösungen mit Hilfe von Natriumsulfat getrocknet. Nach Entfernen des Ethylacetats erhielt man 153,5 g (97 %) brauner Kristalle. Diese wurden aus einem Gemisch von 1000 ml Methanol und 500 ml Wasser umkristallisiert.
Man erhielt 148,7 g (0,94 mol, 94 % der Theorie) 2-Cyano-5,6-cyclohexenopyridinals weiße Kristalle vom Fp: 72 - 74 °C.
$^1$H-NMR ($CDCl_3$): $\delta$ = 7,55-7,31 (AB-System, 2H); 3,07-2,72 (Multiplett, 4H); 2,06-1,71 (Multiplett, 4H)
Analog dieser Vorschriften lassen sich die Verbindungen der folgenden Tabelle 1 herstellen:

| Bsp.Nr. | $R^1$ | $R^2$ | $R^3$ | $R^4$ | Fp [°C] |
|---|---|---|---|---|---|
| 01 | $CH_3$ | H | H | H | 72-73 |
| 02 | H | H | $CH_3$ | H | |
| 03 | $CH_3$ | $CH_3$ | H | H | Öl |
| 04 | $CH_3$ | H | $CH_3$ | H | Öl |
| 05 | $CH_3$ | $CH_3$ | $CH_3$ | H | |
| 06 | $CH_3$ | $CH_3$ | $CH_3$ | $CH_3$ | |
| 07 | $C_6H_5$-$CH_2$ | H | H | H | 84-86 |
| 08 | $C_6H_5$-$CH_2$ | $CH_3$ | H | H | |
| 09 | $C_6H_5$-$CH_2$ | $CH_3$ | $CH_3$ | H | |
| 10 | $C_6H_5$-$CH_2$ | H | $CH_3$ | $CH_3$ | |
| 11 | $C_6H_5$-$CH_2$ | $C_6H_5$-$CH_2$ | H | H | |
| 12 | 4-F-$C_6H_4$-$CH_2$ | H | H | H | |
| 13 | 4-Cl-$C_6H_4$-$CH_2$ | H | H | H | 97-99 |
| 14 | 4-Br-$C_6H_4$-$CH_2$ | H | H | H | |
| 15 | 4-$CH_3$-$C_6H_4$-$CH_2$ | H | H | H | |
| 16 | 4-$CH_3O$-$C_6H_4$-$CH_2$ | H | H | H | |
| 17 | 2-F-$C_6H_4$-$CH_2$ | H | H | H | |
| 18 | 2-Cl-$C_6H_4$-$CH_2$ | H | H | H | |
| 19 | 2,4-$F_2$-$C_6H_3$-$CH_2$ | H | H | H | |
| 20 | 2,4-$Cl_2$-$C_6H_3$-$CH_2$ | H | H | H | |
| 21 | 2-F-4-$CH_3$-$C_6H_3$-$CH_2$ | H | H | H | |
| 22 | $C_2H_5$ | H | H | H | |
| 23 | $C_2H_5$ | $C_2H_5$ | H | H | |
| 24 | $C_2H_5$ | H | $C_2H_5$ | H | |
| 25 | $C_2H_5$ | $C_2H_5$ | $C_2H_5$ | H | |
| 26 | n-$C_4H_9$ | H | H | H | |
| 27 | n-$C_6H_{13}$ | H | H | H | |
| 28 | $C_6H_5$ | H | H | H | |
| 29 | $CH_3$ | H | $C_6H_5$ | H | |
| 30 | H | H | $C_6H_5$-$CH_2$ | H | |
| 31 | H | H | 4-F-$C_6H_4$-$CH_2$ | H | |
| 32 | H | H | 4-Cl-$C_6H_4$-$CH_2$ | H | |

| Bsp.Nr. | $R^1$ | $R^2$ | $R^3$ | $R^4$ | Fp [°C] |
|---|---|---|---|---|---|
| 33 | H | H | $4\text{-}CH_3\text{-}C_6H_4\text{-}CH_2$ | H | |
| 34 | H | H | $4\text{-}CH_3O\text{-}C_6H_4\text{-}CH_2$ | H | |
| 35 | $CH_3$ | H | $C_6H_5\text{-}CH_2$ | H | |
| 36 | $CH_3$ | $CH_3$ | $C_6H_5\text{-}CH_2$ | H | |
| 37 | H | H | $C_6H_5$ | H | 65-66 |
| 38 | H | H | $C_2H_5$ | H | 83-85 |
| 39 | $C_3H_7$ | H | H | H | Öl |
| 40 | $C_3H_7$ | H | $CH_3$ | H | Öl |
| 41 | $4\text{-}Cl\text{-}C_6H_4\text{-}CH_2OCH_2$ | H | H | H | |
| 42 | $CH_3\text{-}OCH_2$ | H | H | H | |
| 43 | $4\text{-}Cl\text{-}C_6H_4$ | H | H | H | |
| 44 | $CH_3\text{-}OCH_2$ | H | $CH_3$ | H | |
| 45 | $CF_3CF_2$ | H | H | H | |
| 46 | $4\text{-}F\text{-}C_6H_4\text{-}CH_2OCH_2$ | H | H | H | |
| 47 | $4\text{-}Cl\text{-}C_6H_4\text{-}CH_2OCH_2$ | $CH_3$ | H | H | |
| 48 | $4\text{-}F\text{-}C_6H_4\text{-}CH_2OCH_2$ | $CH_3$ | H | H | |
| 49 | $4\text{-}F\text{-}C_6H_4$ | $CH_3$ | $CH_3$ | H | |
| 50 | $-CH_2\text{-}CH_2\text{-}CH_2-$ | | H | H | 97-98 |
| 51 | $-CH_2\text{-}CH_2\text{-}CH_2\text{-}CH_2-$ | | H | H | 72-74 |
| 52 | $-CH_2\text{-}CH_2\text{-}CH_2\text{-}CH_2\text{-}CH_2-$ | | H | H | 85-86 |
| 53 | $-CH_2\text{-}CH_2\text{-}CH_2\text{-}CH_2\text{-}CH_2\text{-}CH_2-$ | | H | H | |
| 54 | $-CH_2\text{-}(CH_2\text{-})_6CH_2-$ | | H | H | |
| 55 | $-CH_2\text{-}(CH_2\text{-})_8CH_2-$ | | H | H | 79-80 |
| 56 | $-CH_2\text{-}(CH_2\text{-})_{10}CH_2-$ | | H | H | |
| 57 | $-CH_2\text{-}CH_2\text{-}CH_2\text{-}CH_2-$ | | $CH_3$ | H | |
| 58 | $-CH_2\text{-}CH_2\text{-}CH_2\text{-}CH_2\text{-}CH_2-$ | | $C_6H_5$ | H | |
| 59 | $-CH_2\text{-}CH_2\text{-}CH_2\text{-}CH_2\text{-}CH_2-$ | | $C_6H_5\text{-}CH_2$ | H | |
| 60 | $-CH_2\text{-}CH_2\text{-}CH_2\text{-}CH_2-$ | | $CH_3$ | $CH_3$ | |
| 61 | $-CH_2\text{-}CH_2\text{-}CH_2\text{-}CH_2\text{-}CH_2-$ | | $C_2H_5$ | $C_2H_5$ | |
| 62 | $-CH_2\text{-}CH_2\text{-}CH_2\text{-}CH_2-$ | | $4\text{-}F\text{-}C_6H_4\text{-}CH_2$ | H | |
| 63 | $-CH{=}CH\text{-}CH{=}CH-$ | | H | H | |
| 64 | $-CH{=}CH\text{-}CH{=}CH-$ | | $CH_3$ | H | 79-81 |

| Bsp.Nr. | $R^1$ | $R^2$ | $R^3$ | $R^4$ | Fp [°C] |
|---|---|---|---|---|---|
| 65 | $-CH=CH-CH=CH-$ | | $OCH_3$ | H | |
| 66 | $-CH=CH-C(OCH_3)=CH-$ | | H | H | 177 |
| 67 | $-C(CH_3)=CH-CH=CH-$ | | H | H | 127 |
| 68 | $-CH=CH-CH=CH-$ | | $C_6H_5$ | H | |
| 69 | $-CH=CH-CH=CH-$ | | $CH_3$ | $CH_3$ | |
| 70 | H | H | $-CH=CH-CH=CH-$ | | 85 |
| 71 | $CH_3$ | H | $-CH=CH-CH=CH-$ | | |
| 72 | Cl | H | $-CH=CH-CH=CH-$ | | |
| 73 | H | Cl | $-CH=CH-CH=CH-$ | | |
| 74 | $OCH_3$ | H | $-CH=CH-CH=CH-$ | | |
| 75 | H | $OCH_3$ | $-CH=CH-CH=CH-$ | | |
| 76 | $-CH_2-CH_2-CH_2-CH_2-$ | | H | $CH_3$ | 79-80 |
| 77 | $-C(OCH_3)=CH-CH=CH-$ | | H | H | 105-106 |
| 78 | $CH_3-CH=CH-CH_2-CH_2$ | H | H | H | |
| 79 | $HC \equiv C-(CH_2-)_4$ | H | H | H | |
| 80 | $CH_3-(CH_2-)_3-C \equiv C$ | H | H | H | |

$^1$H-NMR ($CDCl_3$): [$\delta$ in ppm]

s = Singulett; d = Dublett; t = Triplett; q = Quartett, m = Multiplett

Verb.Nr.: 3 $\delta$ = 7,56 (d, 1H); 7,48 (d, 1H); 2,98 (s, 3H); 2,87 (s, 3H)

4 $\delta$ = 7,32 (s, 1H); 7,18 (s, 1H); 2,58 (s, 3H); 2,39 (s, 3H)

22 $\delta$ = 7,79 (t, 1H); 7,51 (d, 1H); 7,41 (d, 1H); 2,87 (q, 2H); 1,31 (t, 3H)

39 $\delta$ = 7,79 (d, 1H); 7,48 (d, 1H); 7,40 (d, 1H); 2,84 (t, 2H); 1,80 (m, 2H); 0,97 (t, 3H)

## Patentansprüche

1. Verfahren zur Herstellung substituierter 2-Cyanopyridine durch Umsetzung entsprechender 1-Alkoxypyridiniumsalze mit Metallcyaniden, dadurch gekennzeichnet, daß man diese in einem polaren aprotischen Lösungsmittel oder einem Gemisch solcher Lösungsmittel miteinander umsetzt.

2. Verfahren gemäß Anspruch 1, dadurch gekennzeichnet, daß man Verbindungen der allgemeinen Formel I herstellt,

(I)

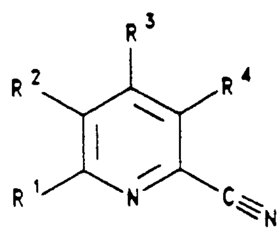

in welcher

$R^1$ H, Alkyl, Alkenyl, Alkinyl, Haloalkyl, Alkoxycarbonyl, Alkoxyalkyl, Phenyl, Benzyl, Phenoxyalkyl bedeutet, wobei die drei letztgenannten Reste im Phenylteil substituiert sein können;

$R^2$ H, Alkyl, Alkenyl, Alkinyl, Haloalkyl oder Alkoxycarbonyl bedeutet;

$R^3$ H, Alkyl, Alkenyl, Alkinyl, Haloalkyl, Alkoxycarbonyl, Phenyl, Phenoxy, Benzyl, wobei die drei letztgenannten Reste substituiert sein können, oder Alkoxy bedeutet; und

$R^4$ H, Alkyl, Alkenyl, Alkinyl, Haloalkyl oder Alkoxycarbonyl bedeutet;

oder

$R^1$ gemeinsam mit $R^2$ für eine gesättigte Kette steht und die übrigen Reste wie oben definiert sind;

oder

$R^1$ gemeinsam mit $R^2$ für eine Kette der Formel -CH=CH-CH=CH- steht, welche substituiert sein kann und die übrigen Reste wie oben definiert sind;

oder

$R^3$ gemeinsam mit $R^4$ für eine Kette der Formel -CH=CH-CH=CH- steht, welche substituiert sein kann und die übrigen Reste wie oben definiert sind;

indem man ein substituiertes 1-Alkoxpyridiniumsalz der allgemeinen Formel II,

(II) 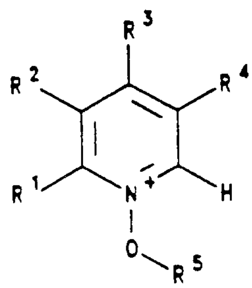 $X^-$

in welcher

$R^1$ bis $R^4$ die gleiche Bedeutung wie in Formel I haben und

$R^5$ Alkyl, Alkenyl, Alkinyl, Benzyl oder Trialkylsilyl bedeutet und

X eine Abgangsgruppe bedeutet;

mit einem Cyanid der allgemeinen Formel III

$M(CN)_m$ (III),

in welcher

M Alkali, Erdalkali oder Kupfer bedeutet und

m 1 oder 2 ist,

in einem polaren aprotischen Lösungsmittel oder Lösungsmittelgemisch miteinander umsetzt.

**3.** Verfahren gemäß Anspruch 1 oder 2, dadurch gekennzeichnet, daß man Verbindungen der Formel I herstellt, in welcher

$R^1$ H, $C_1$-$C_6$-Alkyl, $C_2$-$C_6$-Alkenyl, $C_2$-$C_6$-Alkinyl, $C_1$-$C_4$-Haloalkyl, $C_1$-$C_6$-Alkoxycarbonyl, $C_1$-$C_4$-Alkoxy-$C_1$-$C_4$-alkyl, Phenyl, Benzyl, Phenoxy-$C_1$-$C_2$-alkyl bedeutet, wobei die drei letztgenannten Reste im Phenylteil bis zu fünffach durch gleiche oder verschiedene Reste aus der Reihe $C_1$-$C_4$-Alkyl, Halogen, $C_1$-$C_4$-Alkoxy, $C_1$-$C_4$-Haloalkyl und $C_1$-$C_2$-Haloalkoxy substituiert sein können;

$R^2$ H, $C_1$-$C_6$-Alkyl, $C_2$-$C_6$-Alkenyl, $C_2$-$C_6$-Alkinyl, $C_1$-$C_4$-Haloalkyl oder $C_1$-$C_6$-Alkoxycarbonyl bedeutet;

$R^3$ H, $C_1$-$C_6$-Alkyl, $C_2$-$C_6$-Alkenyl, $C_2$-$C_6$-Alkinyl, $C_1$-$C_4$-Haloalkyl, $C_1$-$C_6$-Alkoxycarbonyl, Phenyl, Phenoxy, Benzyl, wobei die drei letztgenannten Reste bis zu dreifach durch gleiche oder verschiedene Reste aus der Reihe $C_1$-$C_4$-Alkyl, Halogen, $C_1$-$C_4$-Alkoxy, $C_1$-$C_4$-Haloalkyl und $C_1$-$C_2$-Haloalkoxy substituiert sein können, oder $C_1$-$C_4$-Alkoxy bedeutet; und

$R^4$ H, $C_1$-$C_6$-Alkyl, $C_2$-$C_6$-Alkenyl, $C_2$-$C_6$-Alkinyl, $C_1$-$C_4$-Haloalkyl oder $C_1$-$C_6$-Alkoxycarbonyl bedeutet;

oder

$R^1$ gemeinsam mit $R^2$ für $-(CH_2-)_n$ steht, wobei n 2 bis 14 ist und die übrigen Reste wie oben definiert sind;

oder

$R^1$ gemeinsam mit $R^2$ für eine Kette der Formel -CH = CH-CH = CH- steht, welche mit bis zu zwei gleichen oder verschiedenen Resten aus der Reihe $C_1$-$C_4$-Alkyl, Halogen, Phenoxy und $C_1$-$C_4$-Alkoxy substituiert sein kann und die übrigen Reste wie oben definiert sind;

oder

$R^3$ gemeinsam mit $R^4$ für eine Kette der Formel -CH = CH-CH = CH- steht, welche mit bis zu zwei gleichen oder verschiedenen Resten aus der Reihe $C_1$-$C_4$-Alkyl, Halogen und $C_1$-$C_4$-Alkoxy, substituiert sein kann und die übrigen Reste wie oben definiert sind;

indem man ein substituiertes 1-Alkoxypyridiniumsalz der allgemeinen Formel II, in welcher

$R^1$ bis $R^4$ die gleiche Bedeutung wie in Formel I haben und

$R^5$ $C_1$-$C_6$-Alkyl, $C_2$-$C_4$-Alkenyl, $C_2$-$C_4$-Alkinyl, Benzyl oder Tri-$C_1$-$C_6$-alkylsilyl bedeutet und

X eine Abgangsgruppe bedeutet;

umsetzt mit einem Cyanid der allgemeinen Formel III, in welcher

M Alkali, Erdalkali oder Kupfer bedeutet und

m 1 oder 2 ist.

**4.** Verfahren gemäß einem oder mehreren der Ansprüche 1 bis 3, dadurch gekennzeichnet, daß die Abgangsgruppe X ein Rest aus der Reihe Halogen, $C_1$-$C_6$-Alkylsulfonyloxy, $C_6$-$C_{12}$-Arylsulfonyloxy, wobei der letztgenannte Rest im Arylteil bis zweifach durch gleiche oder verschiedene Reste Halogen oder $C_1$-$C_2$-Alkyl substituiert sein kann, $C_1$-$C_4$-Haloalkylsulfonyloxy oder $C_1$-$C_4$-Alkoxysulfonyloxy ist.

**5.** Verfahren gemäß einem oder mehreren der Ansprüche 1 bis 4, dadurch gekennzeichnet, daß man die Umsetzung in Dimethylformamid oder in einem dieses enthaltenden Lösungsmittelgemisch durchführt.

**6.** Verfahren gemäß einem oder mehreren der Ansprüche 1 bis 5, dadurch gekennzeichnet, daß man das Metallcyanid in einem Überschuß von 1 bis 20 mol% bezogen auf das 1-Alkoxypyridiniumsalz einsetzt.

**7.** Verfahren gemäß einem oder mehreren der Ansprüche 1 bis 6, dadurch gekennzeichnet, daß man das Metallcyanid in einem Überschuß von 2 bis 10 mol% bezogen auf das 1-Alkoxypyridiniumsalz einsetzt.

**8.** Verfahren gemäß einem oder mehreren der Ansprüche 1 bis 7, dadurch gekennzeichnet, daß man das Metallcyanid in einem Überschuß von 2 bis 5 mol% bezogen auf das 1-Alkoxypyridiniumsalz einsetzt.

**9.** Verfahren gemäß einem oder mehreren der Ansprüche 1 bis 8, dadurch gekennzeichnet, daß die Reaktionstemperatur für die Umsetzung der Komponenten II und III zwischen -50 °C und +70 °C liegt.

**10.** Verfahren gemäß einem oder mehreren der Ansprüche 1 bis 9, dadurch gekennzeichnet, daß die Reaktionstemperatur für die Umsetzung der Komponenten II und III zwischen -30 °C und +30 °C liegt.

Europäisches Patentamt

# EUROPÄISCHER RECHERCHENBERICHT

Nummer der Anmeldung

EP 93 10 0148

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | JOURNAL OF ORGANIC CHEMISTRY.<br>Bd. 48, 1983, EASTON US<br>Seiten 1375 - 1377<br>W.K. FIFE 'Regioselective cyanation of pyridine 1-oxides with trimethylsilanecarbonitrile: A modified Reissert-Henze reaction.'<br>* das ganze Dokument *<br>--- | 1-10 | C07D213/84<br>C07D215/48<br>C07D217/26<br>C07D221/04 |
| A | EP-A-0 319 254 (SMITHKLINE BECKMAN CORPORATION)<br>* das ganze Dokument *<br>--- | 1-10 | |
| A | EP-A-0 034 349 (TANABE SEIYAKU CO., LTD.)<br>* Seite 38, Zeile 11 - Zeile 22 *<br>--- | 1-10 | |
| A | US-A-4 212 980 (D.E. BUTLER)<br>* das ganze Dokument *<br>----- | 1-10 | |
| | | | RECHERCHIERTE SACHGEBIETE (Int. Cl.5) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 12 MAERZ 1993 | P. BOSMA |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)